# EUROPEAN PATENT SPECIFICATION

(11) **EP 2 833 949 B1**
(45) Date of publication and mention of the grant of the patent: **11.12.2019**
(21) Application number: 13721024.1
(22) Date of filing: 02.04.2013
(51) Int. Cl.: A61B 17/326, A61M 19/00, A61M 5/145, A61M 5/142, A61M 5/42

(54) **CUFF-SHAPED DRUG DELIVERY DEVICE FOR THE PENIS**
MANSCHETTENFÖRMIGES GERÄT ZUR MEDIKAMENTENVERABREICHUNG AM PENIS
APPAREIL DU MODE COUPELLE POUR L'ADMINISTRATION DES MÉDICAMENTS AU PÉNIS

(30) Priority: 30.03.2012 GB 201205638; 16.04.2012 GB 201206665
(43) Date of publication of application: 11.02.2015
(73) Proprietor: Tom Rosenbaum Limited, Richmond, Surrey TW9 4JJ (GB)
(72) Inventor: Rosenbaum, Tomas, Richmond Surrey TW9 4JJ (GB)
(74) Representative: Burrows, Anthony Gregory
(86) International application number: PCT/GB2013/050854
(87) International publication number: WO 2013/144658

(56) References cited:
- WO-A1-98/38944
- WO-A1-2005/051476
- CN-A- 102 188 277
- US-A1- 2002 042 635
- US-A1- 2011 046 556

## Description

This invention relates to an apparatus for the minimally-invasive delivery of a fluid substance into a part of a body.

WO2005/051476 discloses a method for transdermal delivery of a topically applied physiologically active agent comprising providing a micro-projection apparatus comprising an array of fixed microprojections extending from a substrate, applying the array of micro-projections to an area of skin to form an array of microscopic holes therein and contacting the area of skin with a transdermal composition comprising a physiologically active agent and at least one penetration enhancer wherein the formation of the microscopic holes and penetration enhancer facilitate transdermal delivery of the physiologically active agent.

CN102188277 discloses a disposable circumcision anastomat according to the preamble of claim 1, which comprises a fixed knife edge, an internal ring and an external ring, wherein the end part of the knife edge has a medicine outlet. The disposable circumcision anastomat has a medicine injection hole communicated with the medicine outlet.

The medicine injection hole is communicated with the medicine outlet through a channel.

US2011/046556 discloses a local anesthesia injection cone made of a very simple cone shaped piece of clear plastics with a flange on the tip of the cone where a Luer lock syringe can attach. There are multiple fixed tiny hypodermic needles protruding from the base of the cone. The number of needles would be directly proportional to the diameter of the device.

US2002/042635 discloses a non-invasive method and apparatus for delivery of drugs into the penile erectile tissue to induce or enhance penile erection, which affords an alternative treatment for patients afflicted with erectile dysfunction. Sufficient electrical pulses temporarily create new pathways in the penile skin thereby driving a composition, such as a vasoactive or androgenic medication, into the corporal cavernosum.

WO98/38944 dislcoses a self-sealed irrigation system for supplying a treatment fluid to a treatment site. The system includes a flexible containment member that has a delivery channel which delivers treatment fluid to the treatment site, a sealing channel supplied with a suction force, and a recovery channel. The delivery channel delivers the treatment fluid to the treatment site. The recovery channel removes spent treatment fluid from the treatment site. The sealing channel hermetically seals the containment member to the body surface surrounding the area to be treated. The containment member is designed such that any spent treatment solution which should by-pass the recovery channel is drawn into the sealing channel thereby preventing any treatment fluid from leaking to the outside environment. The containment member is connected to a suction pump which circulates treatment fluid through the treatment site, and a reservoir which supplies the treatment fluid to the system.

According to the present invention, there is provided an apparatus according to claim 1 comprising a ring-form device serving to surround a part of a body, a supply of a fluid substance for said device, and a plurality of delivery zones serving to deliver the substance into the body, the arrangement being such that when the fluid substance is placed under an increased pressure it is forced through the plurality of delivery zones to deliver the fluid substance under pressure into the part of the body, wherein each of the plurality of zones comprises a small opening and an area of relative weakness surrounding the opening wherein the area of relative weakness is arranged to bulge under pressure from a first rest position to a second extended position towards a skin surface.

Owing to this aspect, a fluid substance can be delivered relatively quickly in a minimally invasive manner without the use of hypodermic needles.

The device is of a relatively short circumferential length so that pressure generated to force the fluid substance out through the plurality of zones is substantially constant around the whole of the ring-form.

Preferably, the fluid substance is a liquid and advantageously a local anaesthetic substance, although the substance could be any other form of medication that could be delivered with the use of a syringe such as, for example, insulin for diabetics. The apparatus is, however, particularly useful in male circumcision operations where the dorsal nerves in the penis require anaesthetising.

In order that the present invention can be clearly and completely disclosed, reference will now be made, by way of example, to the accompanying drawings, in which :
Figure 1 is a diagrammatic view of a ring-form cuff device for delivering a local anaesthetic to a base of an elongate part of a body,
Figure 2 is a closer view of a part of the cuff device of Figure 1,
Figure 3 is a diagrammatic cross-sectional view of the cuff device against a skin surface according to a first embodiment of the cuff device; and
Figures 4A and 4B are views similar to Figure 3, but of a second embodiment of the cuff device in respective first and second positions.

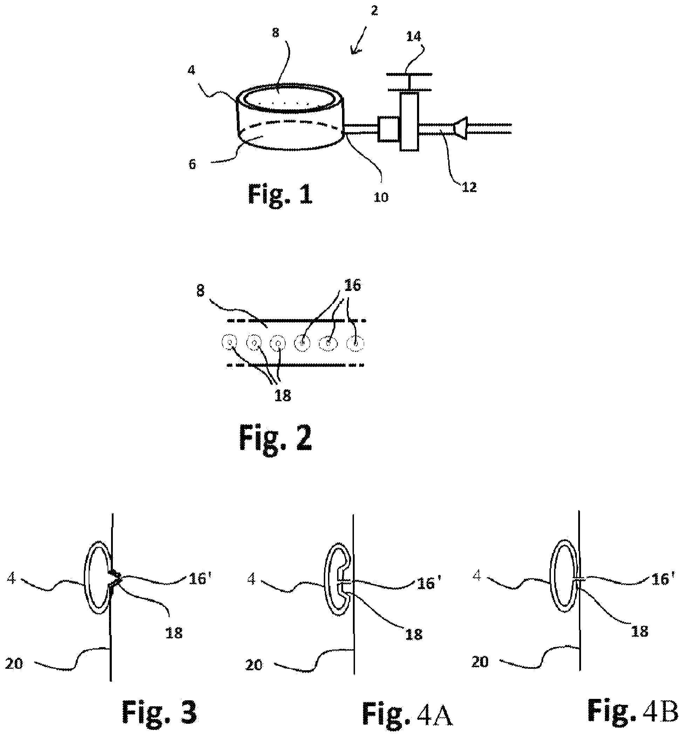

Referring to Figure 1, a fluid delivery device 2 comprises a ring-form cuff device 4 which has an outwardly facing surface 6 and an inwardly facing surface 8. The surface 8 is arranged to lie adjacent a skin surface of, for example, a patient requiring a medicament such as a local anaesthetic, such as in the case of a male human requiring local anaesthetic before a circumcision operation. For such a circumcision operation, the cuff is placed over the end of the patient's penis and located towards the base or root of the shaft of the penis.

The device 2 may have an inlet 10 for connection to a supply 12 of fluid substance, which is preferably an anaesthetic solution, and which can be delivered into the conduit under pressure. A tap 14 may be placed between the source 12 and the inlet 10 to control the delivery of the substance. The source 12 is preferably a syringe containing a suitable amount of the local anaesthetic solution. Alternatively, the supply of the fluid substance could be provided in the cuff itself removing the need for the inlet 10. In this case, a breakable membrane may be needed to prevent leakage of the substance from the cuff 4.

Referring to Figure 2, the surface 8 comprises a plurality of delivery zones 16 arranged in a line around the surface 8. Each delivery zone 16 comprises a small opening 16' in the surface 8 and an area 18 of relative weakness surrounding the opening.

Referring to the version of the cuff 4 shown in Figure 3, the skin 20 around the penis is itself relatively soft and thin owing to the fact that the skin around the penis is fairly loose and elastic. Thus, a local anaesthetic substance subjected to increased pressure in the cuff 4 causes a conical deformation of the area 18 surrounding each zone 16 which thus bulges radially inwardly from a first rest position to a second extended position (as shown) towards the surface of the skin 20. The edges of the areas 18 immediately surrounding the openings 16' thereby contact the skin 20 and, advantageously, break the surface of the skin. The anaesthetic substance is thus delivered into the skin 20 or subcutaneously as desired at each location where an opening is located.

The areas 18 may be formed by a thinning of the material from which the cuff 4 is made or alternatively may be formed from a different material than that of the cuff 4 and suitably attached to the cuff.

Referring to Figure 4, which is a second embodiment of the cuff 4, instead of the plain openings 16' formed in the surface 8, each opening 16' is formed by a small hollow needle 22. In the pre-use state, the delivery zone 16 is at the first rest position (Figure 4A) at which the area of relative weakness 18 is pushed inwardly of the interior of the cuff 4. This ensures that the needle 22 lies in a depressed location. When fluid substance in the cuff 4 is subjected to an increased pressure, the zones 16 are forced outwardly so as to move the zones 16 to the second extended position (Figure 4B) at which the needle 22 pierces the skin surface 20 to deliver the anaesthetic substance subcutaneously where the nerves are.

It is therefore possible to anaesthetise a penis relatively quickly in one step with the very minimal amount of invasiveness rather than delivering a multiple of injections, one at a time, into the penis.

The device 2 is intended to be a single-use disposable item made from medical grade materials, but could also be a re-usable item.

The device 2 can also be used to deliver medicament for post-operative pain that may be experienced by the patient himself.

Although the device 2 has been described in relation to use on a penis, it is also possible that it could be used on other parts of the body requiring treatment. As a result, the device 2 could be of a variety of sizes or be adjustable to different sizes in order to accommodate the variation in size of body parts from person-to-person of similar ages and for all ages of patient. For example, diabetic patients, especially those with a fear of needles, could find this device useful for the administering of insulin into the skin or subcutaneous tissue. Since use of the device is minimally invasive, it would allow for greater freedom of treatment sites on the body to be used, which is particularly useful for diabetics. The delivery of other medicaments is also possible with the present device.

## Claims

1. An apparatus for the delivery of a fluid substance into a part of a body comprising a ring-form device (2) serving to surround a part of the body, a supply (12) of a fluid substance for said device (2), and a plurality of delivery zones (16) serving to deliver the substance into the body, the arrangement being such that when the fluid substance is placed under an increased pressure said fluid substance is forced through the plurality of delivery zones (16) to deliver the fluid substance under pressure into the part of the body, **characterised in that** each of the plurality of the delivery zones (16) comprises a small opening (16') and an area of relative weakness (18) surrounding the opening wherein the area of relative weakness (18) is arranged to bulge under pressure from a first rest position to a second extended position towards a skin surface.

2. An apparatus according to claim 1, wherein the fluid substance is a local anaesthetic substance.

3. An apparatus according to claim 1 or 2, and further comprising an inlet (10) for connection to the supply of the fluid substance.

4. An apparatus according to claim 3, and further comprising a tap (14) located between the supply (12) and the inlet (10) to control the delivery of the fluid substance.

5. An apparatus according to claims 1 or 2, wherein the supply (12) of the fluid substance is provided within the device (2).

6. An apparatus according to claim 5, and further comprising a breakable membrane to prevent leakage of the substance.

7. An apparatus according to any preceding claim, wherein the area of relative weakness (18) is formed by a thinning of the material from which the device (2) is made.

8. An apparatus according to any one of claims 1 to 6, wherein the area of relative weakness (18) is formed from a different material than that of the device (2) and suitably attached to the device.

9. An apparatus according to any preceding claim, wherein each opening (16') is formed by a small hollow needle.

10. An apparatus according to claim 9, wherein when the area of relative weakness (18) is in the first rest position it is pushed inwardly of the interior of the device (2) such that the needle lies in a depressed location and when the fluid substance is subjected to an increased pressure, each zone (16) is forced outwardly so as to move the needle to the second extended position.

## Patentansprüche

1. Vorrichtung zur Abgabe einer fluiden Substanz in einen Teil eines Körpers, umfassend eine ringförmige Vorrichtung (2), die dazu dient, einen Teil des Körpers zu umgeben, einen liefern (12) einer fluiden Substanz für die Vorrichtung (2) und mehrere Abgabezonen (16), die dazu dienen, die Substanz in den Körper abzugeben, wobei die Anordnung so ist, dass, wenn die fluide Substanz einem erhöhten Druck ausgesetzt wird, die fluide Substanz durch die Mehrzahl von Abgabezonen (16) gedrückt wird, um das Fluid abzugeben Substanz unter Druck in den Körperteil, **dadurch gekennzeichnet, dass** jede der Mehrzahl von Abgabezonen (16) eine kleine Öffnung (16') und einen Bereich der relativen Schwäche (18) aufweist, der die Öffnung umgibt, wobei der Bereich der relativen Schwäche ist (18) angeordnet ist, um sich unter Druck von einer ersten Ruheposition zu einer zweiten ausgefahrenen Position in Richtung einer Hautoberfläche zu wölben.

2. Vorrichtung nach Anspruch 1, wobei die fluiden Substanz eine Lokalanästhetika ist.

3. Vorrichtung nach Anspruch 1 oder 2, ferner mit einem Einlaß (10) zur Verbindung mit der Zufuhr der fluiden Substanz.

4. Vorrichtung nach Anspruch 3, ferner mit einem Hahn (14), der zwischen der liefern (12) und dem Einlaß (10) angeordnet ist, um die Abgabe der flüssigen Substanz zu steuern.

5. Vorrichtung nach Anspruch 1 oder 2, bei der der liefern (12) der flüssigen Substanz in der Vorrichtung (2) vorgesehen ist.

6. Vorrichtung nach Anspruch 5, ferner mit eine zerbrechliche Membran, um ein Austreten der Substanz zu verhindern.

7. Vorrichtung nach einem der vorhergehenden Ansprüche, wobei der Bereich der relativen Schwäche (18) durch Verdünnen des Materials gebildet wird, aus dem die Vorrichtung (2) hergestellt ist.

8. Vorrichtung nach einem der Ansprüche 1 bis 6, bei der der Bereich der relativen Schwäche (18) aus einem anderen Material als dem der Vorrichtung (2) gebildet und in geeigneter Weise an der Vorrichtung angebracht ist.

9. Vorrichtung nach einem der vorhergehenden Ansprüche, bei der jede Öffnung (16') durch eine kleine Hohlnadel gebildet wird.

10. Vorrichtung nach Anspruch 9, wobei, wenn sich der Bereich der relativen Schwäche (18) in der ersten Ruheposition befindet, er vom Inneren der Vorrichtung (2) nach innen gedrückt wird, so dass die Nadel an einer vertieften Stelle liegt und sich die fluide Substanz befindet jeder Zone (16) nach außen gedrückt wird, um die Nadel in die zweite ausgefahrene Position zu bewegen.

## Revendications

1. Appareil pour l'administration d'une substance fluide dans une partie d'un corps, comprenant un dispositif en forme d'anneau (2) servant à entourer une partie du corps, une fourniture (12) en une substance fluide pour ledit dispositif (2), et une pluralité de zones de distribution (16) servant à administrer la substance dans le corps, l'agencement étant tel que lorsque la substance fluide est placée sous une pression accrue, ladite substance fluide est forcée à travers la pluralité de zones de distribution (16) pour délivrer le substance fluide sous pression pénétrant dans la partie du corps, **caractérisée en ce que** chacune de la pluralité de zones de distribution (16) comprend une petite ouverture (16') et une zone de faiblesse relative (18) entourant l'ouverture, la zone de faiblesse relative (18) est agencé pour gonfler sous pression d'une première position de repos à une seconde position étendue vers une surface de peau.

2. Appareil selon la revendication 1, dans lequel la substance fluide est une substance anesthésique locale.

3. Appareil selon la revendication 1 ou 2, comprenant en outre une entrée (10) destinée à être connectée à la fourniture de la substance fluide.

4. Appareil selon la revendication 3, comprenant en outre un robinet (14) situé entre la fourniture (12) et l'entrée (10) pour contrôler la distribution de la substance fluide.

5. Appareil selon la revendication 1 ou 2, dans lequel la fourniture (12) de la substance fluide est prévue à l'intérieur du dispositif (2).

6. Appareil selon la revendication 5, comprenant en outre une membrane sécable pour empêcher les fuites de la substance.

7. Appareil selon l'une quelconque des revendications précédentes, dans lequel la zone de faiblesse relative (18) est formée par un amincissement du matériau à partir duquel est fabriqué le dispositif (2).

8. Appareil selon l'une quelconque des revendications 1 à 6, dans lequel la zone de faiblesse relative (18) est formée d'un matériau différent de celui du dispositif (2) et est fixée de manière appropriée au dispositif.

9. Appareil selon l'une quelconque des revendications précédentes, dans lequel chaque ouverture (16') est formée par une petite aiguille creuse.

10. Appareil selon la revendication 9, dans lequel, lorsque la zone de faiblesse relative (18) se trouve dans la première position de repos, elle est poussée vers l'intérieurement de l'intérieur du dispositif (2) de telle sorte que l'aiguille se trouve dans une position en dépression et lorsque la substance fluide est soumis à une pression accrue, chaque zone (16) est forcée vers l'extérieur de manière à déplacer l'aiguille dans la deuxième position étendue.
